(19) Europäisches Patentamt
European Patent Office
Office européen des brevets

(11) EP 1 250 306 B1

(12) EUROPÄISCHE PATENTSCHRIFT

(45) Veröffentlichungstag und Bekanntmachung des Hinweises auf die Patenterteilung:
**09.11.2005 Patentblatt 2005/45**

(21) Anmeldenummer: **01946839.6**

(22) Anmeldetag: **24.01.2001**

(51) Int Cl.[7]: **C07C 51/09**, C07C 51/44, C07C 51/48, B01J 14/00, C07C 53/02

(86) Internationale Anmeldenummer:
**PCT/EP2001/000747**

(87) Internationale Veröffentlichungsnummer:
**WO 2001/055070 (02.08.2001 Gazette 2001/31)**

# (54) VERFAHREN ZUR REINIGUNG VON ABGASSTRÖMEN

METHOD FOR CLEANING OFF-GAS FLOWS

PROCEDE DE PURIFICATION DE FLUX DE FUMEES

(84) Benannte Vertragsstaaten:
**AT BE CH CY DE DK ES FI FR GB GR IE IT LI LU MC NL PT SE TR**

(30) Priorität: **24.01.2000 DE 10002790**

(43) Veröffentlichungstag der Anmeldung:
**23.10.2002 Patentblatt 2002/43**

(73) Patentinhaber: **BASF Aktiengesellschaft**
**67056 Ludwigshafen (DE)**

(72) Erfinder:
- **AUER, Heinz**
  **68809 Neulussheim (DE)**
- **BESSLING, Bernd**
  **67269 Grünstadt (DE)**
- **HAMMER, Hans**
  **68219 Mannheim (DE)**
- **HASSE, Hans**
  **67661 Kaiserslautern (DE)**
- **SAUER, Friedrich**
  **67271 Obersülzen (DE)**
- **VICARI, Maximilian**
  **67117 Limburgerhof (DE)**
- **WAGNER, Gerhard**
  **67069 Ludwigshafen (DE)**
- **ADRIAN, Till**
  **67240 Bobenheim-Roxheim (DE)**

(74) Vertreter: **Isenbruck, Günter et al**
**Isenbruck, Bösl, Hörschler, Wichmann, Huhn**
**Patentanwälte**
**Theodor-Heuss-Anlage 12**
**68165 Mannheim (DE)**

(56) Entgegenhaltungen:
**EP-A- 0 017 866**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 250 306 B1

Printed by Jouve, 75001 PARIS (FR)

## Beschreibung

Die Erfindung betrifft eine Vorrichtung und ein Verfahren zur Gewinnung von wasserfreier oder weitgehend wasserfreier Ameisensäure und die Verwendung des bei dem Verfahren eingesetzten Extraktionsmittels.

Aus "Ullmanns Encyklopädie der technischen Chemie", 4. Auflage, Band 7, Seite 365, ist bekannt, Ameisensäure durch Acydolyse von Formamid mit Schwefelsäure herzustellen. Dieses Verfahren hat jedoch den Nachteil, daß stöchiometrische Mengen Ammoniumsulfat als Zwangsanfall erhalten werden.

Ein weitere Möglichkeit zur Herstellung von Ameisensäure besteht in der Hydrolyse von Methylformiat, das aus Methanol und Kohlenmonoxid synthetisiert wird. Dabei liegen die folgenden Gleichungen zugrunde:

$$CO + CH_3\text{-}OH \rightarrow CH_3\text{-}O\text{-}CO\text{-}H$$
$$\underline{CH_3\text{-}O\text{-}CO\text{-}H + H_2O \rightarrow CH_3\text{-}OH + H\text{-}CO\text{-}OH}$$
$$\text{Brutto:}\quad CO + H_2O \rightarrow H\text{-}CO\text{-}OH$$

Die in "Ullmanns Encyklopädie der technischen Chemie", 4. Auflage, Band 7, Seite 366 beschriebene Hydrolyse von Methylformiat

$$HCOOCH_3 + H_2O \rightleftarrows HCOOH + CH_3OH$$

hat den Nachteil einer ungünstigen Lage des Hydrolysegleichgewichts. Eine Gleichgewichtsverschiebung durch destillative Entfernung der gewünschten Verfahrensprodukte ist nicht möglich, da Methylformiat (Sdp. 32°C) wesentlich niedriger als Methanol (Sdp. 65°C) und Ameisensäure (Sdp. 101°C) siedet. Aus der anfallenden wäßrigen Ameisensäurelösung kann wasserfreie Ameisensäure wegen Azeotropbildung mit Wasser nicht ohne weiteres destillativ gewonnen werden. Die Schwierigkeit besteht also darin, aus dem Hydrolysegemisch des Methylformiats wasserfreie Ameisensäure zu gewinnen.

Ein in der EP-B-0 017 866 beschriebenes, die Verfahrensschritte a) bis g) aufweisendes Verfahren ermöglicht ausgehend von Methylformiat die Herstellung wasserfreier Ameisensäure. Dabei erhält man wasserfreie Ameisensäure, falls man

a) Methylformiat der Hydrolyse unterwirft,
b) vom erhaltenen Hydrolysegemisch Methanol sowie überschüssiges Methylformiat abdestilliert,
c) das Ameisensäure und Wasser enthaltende Sumpfprodukt der Destillation (b) in einer Flüssig-flüssig-Extraktion mit einem hauptsächlich die Ameisensäure aufnehmenden Extraktionsmittel extrahiert,
d) die hierbei erhaltene Ameisensäure, Extraktionsmittel und eine Teilmenge des Wassers aufweisende Extraktphase einer Destillation unterwirft,
e) das bei dieser Destillation erhaltene Wasser und eine Teilmenge der Ameisensäure enthaltende Kopfprodukt in den unteren Teil der Destillationskolonne der Stufe (b) zurückführt,
f) das vorwiegend Extraktionsmittel und Ameisensäure enthaltende Sumpfprodukt der Destillationsstufe (d) destillativ in wasserfreie Ameisensäure und das Extraktionsmittel auftrennt und
g) das die Stufe (f) verlassende Extraktionsmittel in den Verfahrensgang zurückführt.

Es ist bei diesem Verfahren besonders zweckmäßig,

h) die Destillationsschritte (b) und (d) in einer einzigen Kolonne vorzunehmen,
i) das für die Hydrolyse benötigte Wasser dampfförmig in den unteren Teil der für die Durchführung von Schritt (b) vorgesehenen Kolonne einzubringen,
k) Methylformiat und Wasser bei der Hydrolyse (a) im Molverhältnis 1:2 bis 1:10 einzusetzen und/oder
l) als Extraktionsmittel ein Carbonsäureamid der allgemeinen Formel I

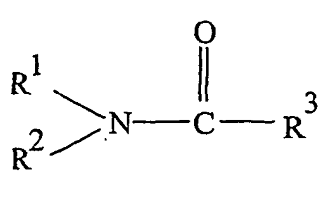

(I)

einzusetzen, in der die Reste $R^1$ und $R^2$ Alkyl-, Cycloalkyl-, Aryl- oder Aralkylgruppen bedeuten oder $R^1$ und $R^2$ gemeinsam zusammen mit dem N-Atom einen heterocyclischen 5- oder 6-Ring ausbilden und daß nur einer der Reste eine Arylgruppe ist und in der $R^3$ für Wasserstoff oder eine $C_1$-$C_4$-Alkylgruppe steht.

Im folgenden werden die Verfahrensschritte (a) bis (i) des vorstehend beschriebenen, aus der EP-B-0 017 866 bekannten Verfahrens näher erläutert.

Verfahrensschritt (a)

Die Hydrolyse wird üblicherweise in einem Temperaturbereich von 80 bis 150°C durchgeführt.

Verfahrensschritt (b)

Die Destillation des Hydrolysegemisches kann prinzipiell bei beliebigem Druck, bevorzugt 0,5 bis 2 bar, vorgenommen werden. Im allgemeinen empfiehlt sich das Arbeiten unter Normaldruck. In diesem Fall beträgt die Temperatur im Kolonnensumpf etwa 110°C und am Kolonnenkopf etwa 30 bis 40°C. Das Hydrolysegemisch wird zweckmäßigerweise in einem Temperaturbereich von 80 bis 150°C zugegebenen, und das Methanol entnimmt man vorzugsweise flüssig bei Temperaturen von 55 bis 65°C. Eine zufriedenstellende Trennung des Gemisches in Methylformiat sowie Methanol einerseits und die wäßrige Ameisensäure andererseits ist bereits mit einer Destillationskolonne möglich, die 25 theoretischen Böden aufweist (bevorzugt,ist eine theoretische Bodenzahl von 35 bis 45).

Die Bauart der für den Verfahrensschritt (b) vorgesehenen Kolonne kann beliebig sein, besonders empfiehlt sich jedoch eine Siebboden- oder Füllkörper-Kolonne.

Verfahrensschritt (c)

Die Flüssig-flüssig-Extraktion der Ameisensäure aus ihrer wäßrigen Lösung mittels eines Extraktionsmittels wird vorzugsweise bei Normaldruck und bei Temperaturen von 60 bis 120, insbesondere 70 bis 90°C im Gegenstrom vorgenommen. Je nach Art des Extraktionsmittels benötigt man in der Regel Extraktionseinrichtungen mit 1 bis 12 theoretischen Trennstufen. Geeignete Extraktionseinrichtungen dafür sind insbesondere Flüssig-flüssig-Extraktionskolonnen. In den meisten Fällen erzielt man mit 4 bis 6 theoretischen Trennstufen befriedigende Ergebnisse.

Die Wahl des Extraktionsmittels ist nicht beschränkt. Als Extraktionsmittel besonders geeignet sind Carbonsäureamide der vorstehend genannten allgemeinen Formel I. Derartige Extraktionsmittel sind vor allem N-Di-n-butylformamid sowie außerdem N-Di-n-butylacetamid, N-Methyl-N-2-heptylformamid, N-n-Butyl-N-2-ethylhexylformamid, N-n-Butyl-N-cyclohexylformamid, und N-Ethylformanilid sowie Gemische dieser Verbindungen. Weitere geeignete Extraktionsmittel sind u.a Diisopropylether, Methylisobutylketon, Ethylacetat, Tributylphosphat und Butandiolformiat.

Verfahrensschritt (d)

Die Extraktphase wird in einer entsprechenden Destillationseinrichtung destillativ in eine Flüssigphase, die in der Regel vorwiegend Ameisensäure und Extraktionsmittel aufweist, sowie eine vorwiegend Wasser und geringe Mengen Ameisensäure aufweisende Dampfphase getrennt. Es handelt sich dabei um eine Extraktivdestillation. Die Sumpftemperatur beträgt vorzugsweise 140 bis 180°C. Ein ausreichender Trenneffekt wird in der Regel ab 5 theoretischen Böden erzielt.

Verfahrensschritt (e)

Die Rückführung des Ameisensäure-Wassergemischs erfolgt in der Regel dampfförmig.

Verfahrensschritte (f) und (g)

Die Destillationseinrichtung (meist als Kolonne ausgebildet) zur Durchführung der Stufe (f) wird zweckmäßigerweise unter vermindertem Druck - etwa 50 bis 300 mbar und entsprechend niedrigen Kopftemperaturen - etwa 30 bis 60°C, betrieben.

Verfahrensschritt (h)

Diese Variante des Verfahrens betrifft die Schritte (b) und (d). Die Destillationseinrichtungen zur Durchführung der Stufen b) und d) werden in einer Gesamtdestillationseinrichtung angeordenet. Die Destillationseinrichtungen sind dabei in der Regel als Kolonnen ausgebildet.

Verfahrensschritt (i)

In diesem Schritt wird für die Hydrolyse benötigtes Wasser in Form von Wasserdampf bereitgestellt.

In dem Verfahren werden insbesondere an den Köpfen der vorhandenen Destillationskolonnen Abgasströme freigesetzt. Diese werden in der Regel über Kondensatoren geleitet, wobei kondensierbare Komponenten auskondensiert und anschließend in das Verfahren zurückgeführt werden. Die an den Kondensatoren nicht auskondensierten, in der Gasphase verbliebenen Komponenten werden als Abgas verworfen bzw. werden der Abgasverbrennung zugeführt. Die Abgasverbrennung ist kostspielig. Außerdem sind in dem Abgasstrom in der Regel noch Wertstoffe, insbesondere Methylformiat, Methanol und/oder Ameisensäure enthalten. Die Verbrennung dieser Wertstoffe verringert die Wirtschaftlichkeit des Verfahrens. Die Ausschleusung von Abgasströmen, die in der Regel neben den vorstehend genannten Wertstoffen noch Inertgase, zum Beispiel Kohlenmonoxid, enthalten, ist aber notwendig, da sich anderenfalls Inertgase in dem Verfahren anreichern würden und die Funktion der Kondensatoren unmöglich wäre.

Aufgabe der vorliegenden Erfindung ist es, ein Verfahren hervorzubringen, bei dem Abgasströme aus dem Verfahren ausgeschleust werden, jedoch die Wertstoffverluste und Entsorgungskosten minimiert werden. Wesentlich ist, daß das Verfahren wirtschaftlich durchführbar ist.

Die Lösung dieser Aufgabe geht aus von dem Verfahren zur Gewinnung von wasserfreier oder weitgehend wasserfreier Ameisensäure, bei dem man,

i) Methylformiat der Hydrolyse unterwirft,
ii) vom erhaltenen Hydrolysegemisch Methanol sowie überschüssiges Methylformiat abdestilliert,
iii) das Ameisensäure und Wasser enthaltende Sumpfprodukt der Destillation ii) in einer Flüssig-flüssig-Extraktion mit einem hauptsächlich die Ameisensäure aufnehmenden Extraktionsmittel extrahiert und dabei als Extraktionsmittel ein Carbonsäureamid der allgemeinen Formel I

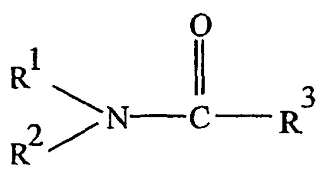

(I)

einsetzt, in der die Reste $R^1$ und $R^2$ Alkyl-, Cycloalkyl-, Aryl- oder Aralkylgruppen bedeuten oder $R_1$ und $R^2$ gemeinsam zusammen mit dem N-Atom einen heterocyclischen 5- oder 6-Ring ausbilden und in der nur einer der Reste eine Arylgruppe ist und in der $R^3$ für Wasserstoff oder eine $C_1$-$C_4$-Alkylgruppe steht.
iv) die hierbei erhaltene, Ameisensäure, Extraktionsmittel und eine Teilmenge des Wassers aufweisende Extraktphase einer Destillation unterwirft,
v) das bei dieser Destillation erhaltene, Wasser und eine Teilmenge der Ameisensäure enthaltende Kopfprodukt in den unteren Teil der Destillationseinrichtung der Stufe ii) zurückführt,
vi) das vorwiegend Extraktionsmittel und Ameisensäure enthaltende Sumpfprodukt der Destillationsstufe iv) destillativ in wasserfreie oder weitgehend wasserfreie Ameisensäure und das Extraktionsmittel auftrennt und
vii) das die Stufe vi) verlassende Extraktionsmittel in den Verfahrensgang zurückführt.

Das erfindungsgemäße Verfahren ist dann dadurch gekennzeichnet, daß man in dem Prozeß anfallende, Methylformiat und/oder Methanol und/oder Ameisensäure enthaltende gasförmige Abgasströme vor dem Ausschleusen aus dem Prozeß mit dem eingesetzten Extraktionsmittel wäscht, wobei sich Methylformiat und/oder Methanol und/oder Ameisensäure in dem Extraktionsmittel lösen und das mit Methylformiat und/oder Methanol und/oder Ameisensäure beladene Extraktionsmittel wieder in den Prozeß zurückgeführt wird.

In dem Verfahren können ein oder mehrere Abgasströme mit dem Extraktionsmittel gewaschen werden. Vorteilhaft ist es, Abgasströme zu vereinigen und anschließend der Wäsche zu unterziehen. Es können jedoch auch in die für die Abgaswäsche vorgesehene Abgaswascheinrichtung mehrere Abgasströme eingeführt werden.

Die mit Extraktionsmittel ausgewaschenen Abgasströme können vorteilhafterweise ohne anschließende Abgasverbrennung entsorgt werden. Dies erspart erhebliche Kosten. Auch durch die Rückführung der Wertstoffe, insbesondere Methylformiat und/oder Methanol und/oder Ameisensäure können erhebliche Kosten eingespart werden. Wesentlich ist es, daß das zur Wäsche eingesetzte Lösungsmittel gleichzeitig als Extraktionsmittel für die Ameisensäure und als Waschflüssigkeit für das Abgas eingesetzt wird - für die Durchführung der Abgaswäsche muß somit kein weiterer zusätzlicher Hilfsstoff in den Prozeß eingeführt werden. Dies ist von erheblicher wirtschaftlicher Bedeutung.

Die für das Verfahren eingesetzten Extraktionsmittel sind relativ schwerflüchtig und können somit nicht von

dem zu reinigenden Abgasstrom ausgestrippt werden.

Als Extraktionsmittel werden bevorzugt N,N-Di-n-butylformamid, N,N-Di-n-butylacetamid, N-Methyl-N-2-heptylformamid, N-n-Butyl-N-2-ethylhexylformamid, N-n-Butyl-N-cyclohexylformamid und/oder N-Ethylformanilid eingesetzt. Es besteht sowohl die Möglichkeit nur ein Extraktionsmittel als auch ein Gemisch, das mehrere Extraktionsmittel enthält, einzusetzen. Das zur Wäsche eingesetzte Extraktionsmittel kann neben dem "eigentlichen Extraktionsmittel" auch noch andere Komponenten enthalten, insbesondere Ameisensäure. Das Extraktionsmittel wird der Abgaswascheinrichtung meist mit einer Temperatur zwischen 10 und 60°C, insbesondere zwischen 20 und 50°C, zugeführt.

In der Regel fallen die mit dem eingesetzten Extraktionsmittel gewaschenen Abgasströme in Schritt ii) und/oder Schritt vi) des erfindungsgemäßen Verfahrens an. Dabei werden die Abgasströme in der Regel am Kopf der entsprechenden Destillationskolonnen entnommen und Kondensatoren zugeführt. Vor der Wäsche werden dann entsprechend in dem Abgasstrom enthaltene kondensierbare Komponenten durch Kondensation teilweise aus den Abgasströmen entfernt. Die auskondensierten Komponenten werden dann in der Regel in das Verfahren zurückgeführt, während das von den auskondensierten Komponenten befreite Abgas der Wäsche zugeführt wird. Unter kondensierbaren Komponenten sollen in diesem Zusammenhang Komponenten verstanden werden wie Methanol, Ameisensäure oder Methylformiat, die sich bei 0°C und Atmosphärendruck auskondensieren lassen - Inertgase wie Stickstoff oder Kohlenmonoxid sollen als nichtkondensierbare Komponenten angesehen werden, da diese vollständig im Abgasstrom verbleiben. Hingegen werden kondensierbare Komponenten in der Regel nur teilweise kondensiert. Die nicht kondensierten Anteile gelangen in den Abgasstrom, der mit dem Extraktionsmittel gewaschen wird.

Das nach der Wäsche mit Methylformiat und/oder Methanol und/oder Ameisensäure beladene Extraktionsmittel wird meist in Schritt iii) und/oder Schritt iv) wieder in den Prozeß zurückgeführt - dies entspricht in der Regel der Rückführung in die Extraktionseinrichtung und/oder in die Destillationseinrichtung zur Durchführung der Stufe vi).

In der Regel wird das zur Wäsche eingesetzte Extraktionsmittel als Teilstrom dem die Stufe vi) verlassenden Extraktionsmittel und/oder der die Stufe iii) verlassenden, im wesentlichen Ameisensäure und Extraktionsmittel enthaltenden Extraktphase, entnommen.

Die Wäsche des Abgases mit dem Extraktionsmittel erfolgt in der Regel in einer Abgaswascheinrichtung.

Die Erfindung betrifft außerdem die Verwendung eines Carbonsäureamids der allgemeinen Formel I

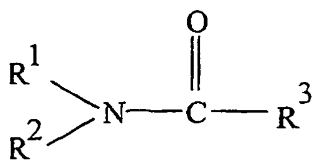

(I)

in der die Reste $R^1$ und $R^2$ Alkyl-, Cycloalkyl-, Aryl- oder Aralkylgruppen bedeuten oder $R^1$ und $R^2$ gemeinsam zusammen mit dem N-Atom einen heterocyclischen 5- oder 6-Ring ausbilden und in der nur einer der Reste eine Arylgruppe ist und in der $R^3$ für Wasserstoff oder eine $C_1$-$C_4$-Alkylgruppe steht, in dem vorstehend beschriebenen Verfahren als Extraktionsmittel für die Flüssig-flüssig-Extraktion von Ameisensäure und als Waschflüssigkeit für Methylformiat und/oder Methanol und/oder Ameisensäure enthaltende gasförmige Abgasströme.

Erfindungsgemäß wird auch eine Vorrichtung zur Durchführung des vorstehend erläuterten Verfahrens bereitgestellt. Diese enthält

α) einen Synthesereaktor,
β) einen Hydrolysereaktor,
χ) eine Destillationseinrichtung zur Durchführung der Stufe ii),
δ) eine Destillationseinrichtung zur Durchführung der Stufe iv),
ε) eine Extraktionseinrichtung,
φ) eine Destillationseinrichtung zur Durchführung der Stufe vi) und
γ) eine Abgaswascheinrichtung.

Als Synthesereaktor soll eine Einrichtung verstanden werden, in der einerseits die Synthese von Methylformiat erfolgt (meist in einem entsprechenden Reaktor) und in der gegebenenfalls andererseits noch eine Trennung des erhaltenen Synthesegemischs (meist in einer dem Reaktor nachgeschalteten Destillationseinrichtung) durchgeführt wird. Als Hydrolysereaktor können beliebige Reaktoren eingesetzt werden, mit der die Hydrolyse von Methylformiat durchgeführt werden kann. Als Destillationseinrichtungen kommen insbesondere Destillationskolonnen in Frage. Für die Extraktion der Ameisensäure wird insbesondere eine Extraktionseinrichtung eingesetzt, die als Flüssig-flüssig-Extraktionskolonne ausgebildet ist. Für die Durchführung der Abgaswäsche wird in der Regel eine Abgaswascheinrichtung eingesetzt, welche bevorzugt als Waschkolonne ausgestaltet ist. Diese enthält meist 3 bis 20, insbesonders 4 bis 10, theoretische Trennstufen.

In einer bevorzugten Ausführungsform der Erfindung sind die Destillationseinrichtung zur Durchführung der Stufe ii) und die Destillationseinrichtung zur Durchführung der Stufe iv) in einer einzigen Destillationseinrichtung angeordnet. Letzere ist in der Regel als Destillationskolonne ausgebildet.

Die anliegende Zeichnung zeigt

| | |
|---|---|
| in Figur 1 und in Figur 2 | Schemata von Anlagen zur Durchführung des Verfahrens nach dem Stand der Technik sowie |
| in Figur 3 und in Figur 4 | Schemata von Anlagen zur Durchführung des erfindungsgemäßen Verfahrens. |

Die mit Pfeilen gekennzeichneten Ströme sind zum Teil mit Bezugszeichen versehen. Diese geben die in diesen Strömen enthaltenen Komponenten an, die darin in der Regel den größten Anteil aufweisen. Da die Anteile der Komponenten in den Strömen variieren können, dienen diese Bezugszeichen nur als Richtwerte. Dabei bedeuten 21 Methylformiat, 22 Wasser, 23 Ameisensäure, 24 Methanol, 25 Extraktionsmittel und 27 Kohlenmonoxid.

Den Anlagen zur Durchführung des Verfahrens nach dem Stand der Technik (Fig. 1, Fig. 2) und den Anlagen zur Durchführung des erfindungsgemäßen Verfahrens (Fig. 3, Fig. 4) ist gemeinsam, daß diese einen Synthesereaktor 6, einen Hydrolysereaktor 1, eine Destillationseinrichtung 2 zur Durchführung der Stufe ii), eine Destillationseinrichtung 4 zur Durchführung der Stufe iv), eine Extraktionseinrichtung 3 zur Durchführung der Stufe iii) und eine Destillationseinrichtung 5 zur Durchführung der Stufe vi) aufweisen. Dabei können die Destillationseinrichtungen 2; 4 in einer einzigen Destillationseinrichtung 7 angeordnet sein.

Im Gegensatz zu den in Fig. 1 und Fig. 2 aufgezeigten Anlagen weisen die in Fig. 3 und Fig. 4 dargestellten Anlagen zur Durchführung des erfindungsgemäßen Verfahrens eine Vakuumpumpe 8, eine Gaswascheinrichtung 9 und eine Leitung 13 für die Zuführung von Extraktionsmittel in die Gaswascheinrichtung 9 auf. Bei den Anlagen gemäß dem Stand der Technik (Fig. 1, Fig. 2) verläßt das Abgas den Prozeß über eine Leitung 12, welche hinter Kondensatoren angeordnet ist. Im Gegensatz dazu ist bei den Anlagen gemäß der Erfindung (Fig. 3, Fig. 4) die Leitung 12, durch die Abgas aus dem Verfahren ausgeschleust wird, der Abgaswascheinrichtung 9 nachgeschaltet.

Im folgenden soll die Erfindung anhand eines Ausführungsbeispiels näher erläutert werden.

**Beispiel**

Dem Beispiel liegt ein erfindungsgemäßes Verfahren zugrunde, das in einer Anlage durchgeführt wird, die schematisch in Fig. 4 aufgezeigt ist. In der Anlage werden kontinuierlich 5,3 kg/h wäßrige Ameisensäure hergestellt. Dabei fallen 94,7 1/h Abgas an, welches in einer Abgaswascheinrichtung 9 mit 1,1 kg/h des Extraktionsmittels N,N-Di-n-Butylformamid von kondensierbaren Komponenten (Ameisensäure, Methylformiat, Methanol) befreit wird. Die als Abgaswascheinrichtung eingesetzte Laborkolonne weist einen Durchmesser von 30 mm auf und ist mit 30 Glockenböden ausgestattet.

Die Ergebnisse des Versuchs sind in der nachstehenden Tabelle 1 aufgeführt.

Tabelle 1

| | **Ameisensäure** | **Abgas (ungereinigt)** | **Abgas (gereinigt)** | **Extraktionsmittel (unbeladen)** | **Extraktionsmittel (beladen)** |
|---|---|---|---|---|---|
| Anfall pro Zeiteinheit kg/h | 5,300 | 0,176 | 0,082 | 1,113 | 1,207 |
| Wasser Zusammensetzung Gew.-% | 5,7 | 0 | 0 | 0 | 0 |
| Extraktionsmittel Zusammensetzung Gew.-% | 0 | 0 | 0 | 99,1 | 91,4 |
| Ameisensäure Zusammensetzung Gew.-% | 94,3 | 3,9 | 0 | 0,9 | 1,4 |

Tabelle 1 (fortgesetzt)

| | Ameisensäure | Abgas (ungereinigt) | Abgas (gereinigt) | Extraktionsmittel (unbeladen) | Extraktionsmittel (beladen) |
|---|---|---|---|---|---|
| Methylformiat Zusammensetzung Gew.-% | 0 | 49,4 | 1,8 | - | 7,1 |
| Methanol Zusammensetzung Gew.-% | 0 | 1,1 | 0 | - | 0,2 |
| Inertgas Zusammensetzung Gew.-% | 0 | 45,6 | 98,2 | -- | 0 |
| Temperatur °C | -- | 47 | 31 | 30 | 46 |

Die Versuchsergebnisse zeigen, daß das Abgas nach der Wäsche weitgehend von den Wertstoffen Ameisensäure, Methylformiat und Methanol befreit ist. Das gereinigte Abgas enthält nur noch 1,8 Gew.-% Wertstoffe, während das ungereinigte Abgas insgesamt 54, 4 Gew.-% Wertstoffe enthält. Es zeigt sich, daß sich das eingesetzte Extraktionsmittel N,N-Di-n-Butylformamid als Waschflüssigkeit für die in dem Verfahren freigesetzten Abgase eignet.

## Patentansprüche

**1.** Verfahren zur Gewinnung von wasserfreier oder weitgehend wasserfreier Ameisensäure bei dem man,

i) Methylformiat der Hydrolyse unterwirft,
ii) vom erhaltenen Hydrolysegemisch Methanol sowie überschüssiges Methylformiat abdestilliert,
iii) das Ameisensäure und Wasser enthaltende Sumpfprodukt der Destillation ii) in einer Flüssig-flüssig-Extraktion mit einem hauptsächlich die Ameisensäure aufnehmenden Extraktionsmittel extrahiert und dabei als Extraktionsmittel ein Carbonsäureamid der allgemeinen Formel I

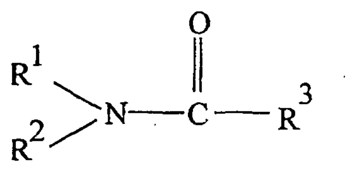

I

einsetzt, in der die Reste $R^1$ und $R^2$ Alkyl-, Cycloalkyl-, Aryl- oder Aralkylgruppen bedeuten oder $R^1$ und $R^2$ gemeinsam zusammen mit dem N-Atom einen heterocyclischen 5- oder 6-Ring ausbilden und in der nur einer der Reste eine Arylgruppe ist und in der $R^3$ für Wasserstoff oder eine $C_1$-$C_4$-Alkylgruppe steht.
iv) die hierbei erhaltene, Ameisensäure, Extraktionsmittel und eine Teilmenge des Wassers aufweisende Extraktphase einer Destillation unterwirft,
v) das bei dieser Destillation erhaltene, Wasser und eine Teilmenge der Ameisensäure enthaltende Kopfprodukt in den unteren Teil der Destillationskolonne der Stufe ii) zurückführt,
vi) das vorwiegend Extraktionsmittel und Ameisensäure enthaltende Sumpfprodukt der Destillationsstufe iv) destillativ in wasserfreie bzw. weitgehend wasserfreie Ameisensäure und das Extraktionsmittel auftrennt und
vii) das die Stufe vi) verlassende Extraktionsmittel in den Verfahrensgang zurückführt,

**dadurch gekennzeichnet, daß** man in dem Prozeß anfallende, Methylformiat und/oder Methanol und/oder Ameisensäure enthaltende gasförmige Abgasströme vor dem Ausschleusen aus dem Prozeß mit dem eingesetzten Extraktionsmittel wäscht, wobei sich Methylformiat und/oder Methanol und/oder Ameisensäure in dem Extraktionsmittel lösen und das mit Methylformiat und/oder Methanol und/oder Ameisensäure beladene Extraktionsmittel wieder in den Prozeß zurückgeführt wird.

2. Verfahren nach Anspruch 1, **dadurch gekennzeichnet, daß** als Extraktionsmittel N,N-Di-n-butylformamid, N,N-Di-n-butylacetamid, N-Methyl-N-2-heptylformamid, N-n-Butyl-N-2-ethylhexylformamid, N-n-Butyl-N-cyclohexylformamid und/oder N-Ethylformanilid eingesetzt wird.

3. Verfahren nach Anspruch 1 oder 2, **dadurch gekennzeichnet, daß** das mit Methylformiat und/oder Methanol und/oder Ameisensäure beladene Extaktionsmittel in Schritt iii) und/oder Schritt iv) wieder in den Prozeß zurückgeführt wird.

4. Verfahren nach einem der Ansprüche 1 bis 3, **dadurch gekennzeichnet, daß** die mit dem eingesetzten Extraktionsmittel gewaschenen Abgasströme in Schritt ii) und/oder Schritt vi) anfallen und vor der Wäsche kondensierbare Komponenten durch Kondensation aus den Abgasströmen entfernt werden.

5. Verfahren nach einem der Ansprüche 1 bis 4, **dadurch gekennzeichnet, daß** das zur Wäsche eingesetzte Extraktionsmittel als Teilstrom dem die Stufe vi) verlassenden Extraktionsmittel und/oder der die Stufe iii) verlassenden, im wesentlichen Ameisensäure und Extraktionsmittel enthaltenden Extraktphase, entnommen wird.

6. Verfahren nach einem der Ansprüche 1 bis 5, **dadurch gekennzeichnet, daß** die Destillationsschritte ii) und iv) in einer einzigen Destillationseinrichtung durchgeführt werden.

7. Verwendung eines Carbonsäureamids der allgemeinen Formel I

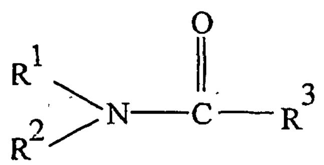

I

in der die Reste $R^1$ und $R^2$ Alkyl-,Cycloalkyl-, Aryl- oder Aralkylgruppen bedeuten oder $R^1$ und $R^2$ gemeinsam zusammen mit dem N-Atom einen heterocyclischen 5- oder 6-Ring ausbilden und daß nur einer der Reste eine Arylgruppe ist und in der $R^3$ für Wasserstoff oder eine $C_1$-$C_4$-Alkylgruppe steht, in dem Verfahren gemäß einem der Ansprüche 1 bis 6 als Extraktionsmittel für die Flüssig-flüssig-Extraktion von Ameisensäure und als Waschflüssigkeit für Methylformiat und/oder Methanol und/oder Ameisensäure enthaltende gasförmige Abgasströme.

8. Vorrichtung zur Durchführung des Verfahrens gemäß eines der Ansprüche 1 bis 6, enthaltend

α) einen Synthesereaktor (6),
β) einen Hydrolysereaktor (1),
χ) eine Destillationseinrichtung (2) zur Durchführung der Stufe ii),
δ) eine Destillationseinrichtung (4) zur Durchführung der Stufe iv),
ε) eine Extraktionseinrichtung (3),
ϕ) eine Destillationseinrichtung (5) zur Durchführung der Stufe vi) und
γ) eine Abgaswascheinrichtung (9).

9. Vorrichtung nach Anspruch 8, **dadurch gekennzeichnet, daß** die Destillationseinrichtung (2) zur Durchführung der Stufe ii) und die Destillationseinrichtung (4) zur Durchführung der Stufe iv) in einer einzigen Destillationseinrichtung (7) angeordnet sind.

## Claims

1. A process for obtaining anhydrous or substantially anhydrous formic acid, in which

i) methyl formate is subjected to hydrolysis,
ii) methanol and excess methyl formate are distilled off from the resultant hydrolysis mixture,
iii) the bottom product from distillation ii), comprising formic acid and water, is extracted in a liquid-liquid extraction with an extractant which principally takes up the formic acid, and the extractant employed here is a

carboxamide of the general formula I

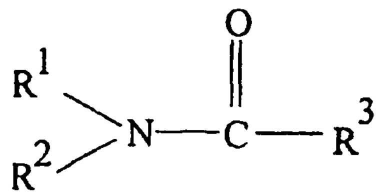

(I)

where the radicals $R^1$ and $R^2$ are alkyl, cycloalkyl, aryl or aralkyl groups, or $R^1$ and $R^2$ jointly, together with the N atom, form a heterocyclic 5- or 6-membered ring, and where only one of the radicals is an aryl group, and where $R^3$ is hydrogen or a $C_1$-$C_4$-alkyl group,
iv) the resultant extract phase, comprising formic acid, extractant and some of the water, is subjected to distillation,
v) the top product obtained in this distillation, which comprises water and some of the formic acid, is fed back into the lower part of the distillation device in step ii),
vi) the bottom product from distillation step iv), which comprises predominantly extractant and formic acid, is separated by distillation into anhydrous or substantially anhydrous formic acid and the extractant, and
vii) the extractant leaving step vi) is fed back into the process,

which comprises washing gaseous offgas streams arising in the process and comprising methyl formate and/or methanol and/or formic acid with the extractant employed before discharge from the process, during which methyl formate and/or methanol and/or formic acid dissolve in the extractant, and the extractant loaded with methyl formate and/or methanol and/or formic acid is fed back into the process.

2. The process according to claim 1, wherein the extractant employed is N,N-di-n-butylformamide, N,N-di-n-butylacetamide, N-methyl-N-2-heptylformamide, N-n-butyl-N-2-ethylhexylformamide, N-n-butyl-N-cyclohexylformamide and/or N-ethylformanilide.

3. The process according to claim 1 or 2, wherein the extractant loaded with methyl formate and/or methanol and/or formic acid is fed back into the process in step iii) and/or step iv).

4. The process according to one of claims 1 to 3, wherein the offgas streams washed with the extractant employed are produced in step ii) and/or step vi), and condensable components are removed from the offgas streams by condensation before the washing.

5. The process according to one of claims 1 to 4, wherein the extractant employed for the washing is taken as a substream from the extractant leaving step vi) and/or the extract phase leaving step iii), which essentially comprises formic acid and extractant.

6. The process according to one of claims 1 to 5, wherein distillation steps ii) and iv) are carried out in a single distillation device.

7. The use of a carboxamide of the general formula I

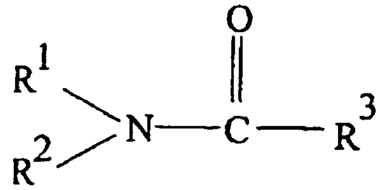

(I)

where the radicals $R^1$ and $R^2$ are alkyl, cycloalkyl, aryl or aralkyl groups, or $R^1$ and $R^2$ jointly, together with the N atom, form a heterocyclic 5- or 6-membered ring, and only one of the radicals is an aryl group, and where $R^3$ is hydrogen or a $C_1$-$C_4$-alkyl group, in the process according to one of claims 1 to 6 as extractant for the liquid-liquid extraction of formic acid and as washing liquid for gaseous offgas streams comprising methyl formate and/or methanol and/or formic acid.

**8.** An apparatus for carrying out the process according to one of claims 1 to 6, comprising

α) a synthesis reactor (6),
β) a hydrolysis reactor (1),
χ) a distillation device (2) for carrying out step ii),
δ) a distillation device (4) for carrying out step iv),
ε) an extraction device (3),
ϕ) a distillation device (5) for carrying out step vi), and
γ) an offgas washing device (9).

**9.** The apparatus according to claim 8, wherein the distillation device (2) for carrying out step ii) and the distillation device (4) for carrying out step iv) are arranged in a single distillation device (7).

**Revendications**

**1.** Procédé de production d'acide formique anhydre ou profondément anhydre, dans lequel

i) on soumet du formiate de méthyle à l'hydrolyse,
ii) on sépare du méthanol ainsi que du formiate de méthyle excédentaire par distillation du mélange d'hydrolyse obtenu,
iii) on extrait le produit de fond de cuve de la distillation ii), qui contient de l'acide formique et de l'eau, dans une extraction liquide-liquide avec un agent d'extraction reprenant principalement l'acide formique, et on met en oeuvre, comme agent d'extraction, un amide d'acide carboxylique de la formule générale I :

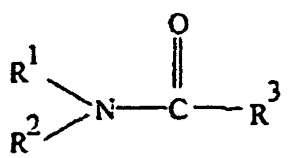

I

dans laquelle les radicaux $R^1$ et $R^2$ représentent des groupes alkyle, cycloalkyle, aryle ou aralkyle ou $R^1$ et $R^2$ forment conjointement avec l'atome de N un noyau hétérocyclique pentagonal ou hexagonal, seul un des radicaux étant un groupe aryle et $R^3$ représentant de l'hydrogène ou un groupe alkyle en $C_1$-$C_4$,
iv) on soumet à une distillation la phase d'extrait obtenue qui présente de l'acide formique, de l'agent d'extraction et une quantité partielle de l'eau,
v) on recycle le produit de tête obtenu dans cette distillation, qui contient de l'eau et une quantité partielle de l'acide formique, dans la partie inférieure de la colonne de distillation de l'étape ii),
vi) on sépare par distillation le produit de fond de cuve de l'étape de distillation iv) qui contient principalement de l'agent d'extraction et de l'acide formique en acide formique anhydre ou respectivement profondément anhydre et l'agent d'extraction, et
vii) on recycle l'agent d'extraction quittant l'étape vi) dans le processus,

**caractérisé en ce qu'**on lave avec l'agent d'extraction mis en oeuvre des courants de gaz résiduaire obtenus dans le processus, qui contiennent du formiate de méthyle et/ou du méthanol et/ou de l'acide formique, avant l'éclusage hors du processus, du formiate de méthyle et/ou du méthanol et/ou de l'acide formique se dissolvant dans l'agent d'extraction, l'agent d'extraction chargé de formiate de méthyle et/ou de méthanol et/ou d'acide formique étant recyclé à nouveau dans le processus.

**2.** Procédé suivant la revendication 1, **caractérisé en ce que**, comme agent d'extraction, on met en oeuvre du N,N-di-n-butylformamide, du N,N-di-n-butylacétamide, du N-méthyl-N-2-heptylformamide, du N-n-butyl-N-2-éthylhexylformamide, du N-n-butyl-N-cyclohexylformamide et/ou du N-éthylformanilide.

**3.** Procédé suivant la revendication 1 ou 2, **caractérisé en ce que** l'agent d'extraction chargé de formiate de méthyle et/ou de méthanol et/ou d'acide formique est recyclé à nouveau dans le processus dans l'étape iii) et/ou iv).

**4.** Procédé suivant l'une des revendications 1 à 3, **caractérisé en ce que** les courants de gaz résiduaire lavés par l'agent d'extraction mis en oeuvre sont obtenus dans l'étape ii) et/ou vi) et **en ce que** des composants condensables sont éliminés par condensation hors des courants de gaz résiduaire, avant le lavage.

**5.** Procédé suivant l'une des revendications 1 à 4, **caractérisé en ce que** l'agent d'extraction mis en oeuvre pour le lavage est prélevé, comme courant partiel, de l'agent d'extraction quittant l'étape vi) et/ou de la phase d'extrait quittant l'étape iii), qui contient essentiellement de l'acide formique et de l'agent d'extraction.

**6.** Procédé suivant l'une des revendications 1 à 5, **caractérisé en ce que** les étapes de distillation ii) et iv) sont effectuées dans un dispositif de distillation unique.

**7.** Utilisation d'un amide d'acide carboxylique de la formule générale I :

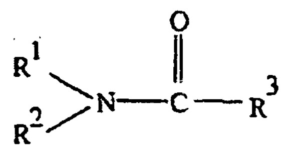

I

dans laquelle les radicaux $R^1$ et $R^2$ représentent des groupes alkyle, cycloalkyle, aryle ou aralkyle, ou $R^1$ et $R^2$ forment conjointement avec l'atome de N un noyau hétérocyclique pentagonal ou hexagonal, seul un des radicaux étant un groupe aryle et $R^3$ représentant de l'hydrogène ou un groupe alkyle en $C_1$-$C_4$, dans le procédé suivant l'une des revendications 1 à 6, comme agent d'extraction pour l'extraction liquide-liquide d'acide formique et comme liquide de lavage pour des courants de gaz résiduaire contenant du formiate de méthyle et/ou de méthanol et/ou de l'acide formique.

**8.** Dispositif pour la mise en oeuvre du procédé suivant l'une des revendications 1 à 6, comprenant

α) un réacteur de synthèse (6),
β) un réacteur d'hydrolyse (1),
χ) un dispositif de distillation (2) pour la mise en oeuvre de l'étape ii),
δ) un dispositif de distillation (4) pour la mise en oeuvre de l'étape iv),
ε) un dispositif d'extraction (3),
ϕ) un dispositif de distillation (5) pour la mise en oeuvre de l'étape vi), et
γ) un dispositif de lavage de gaz résiduaire (9).

**9.** Dispositif suivant la revendication 8, **caractérisé en ce que** le dispositif de distillation (2) pour la mise en oeuvre de l'étape ii) et le dispositif de distillation (4) pour la mise en oeuvre de l'étape iv) sont agencés dans un dispositif de distillation unique (7).

FIG.1

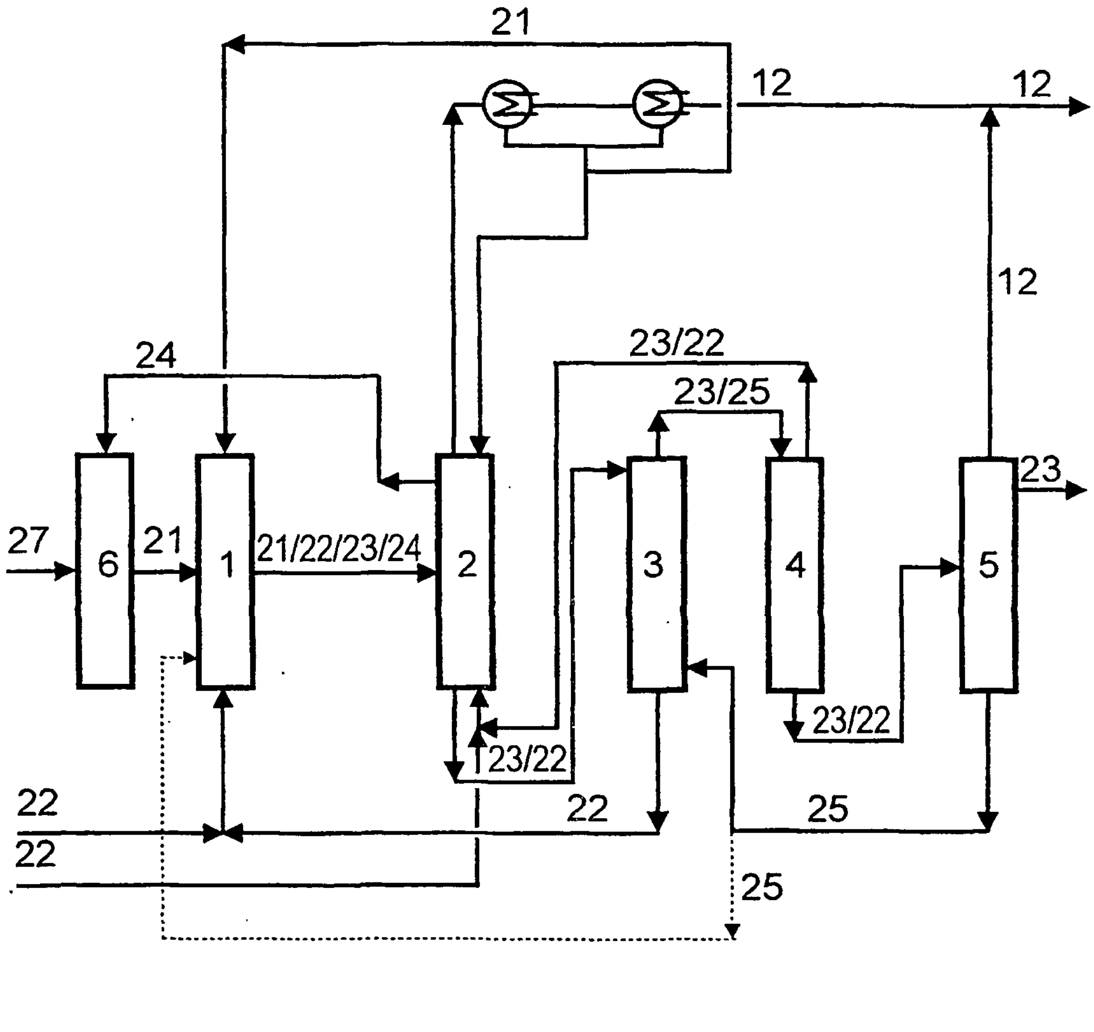
21
12
12
12
24
23/22
23/25
27
6
21
1
21/22/23/24
2
3
4
5
23
23/22
23/22
22
22
22
25
25

FIG.2

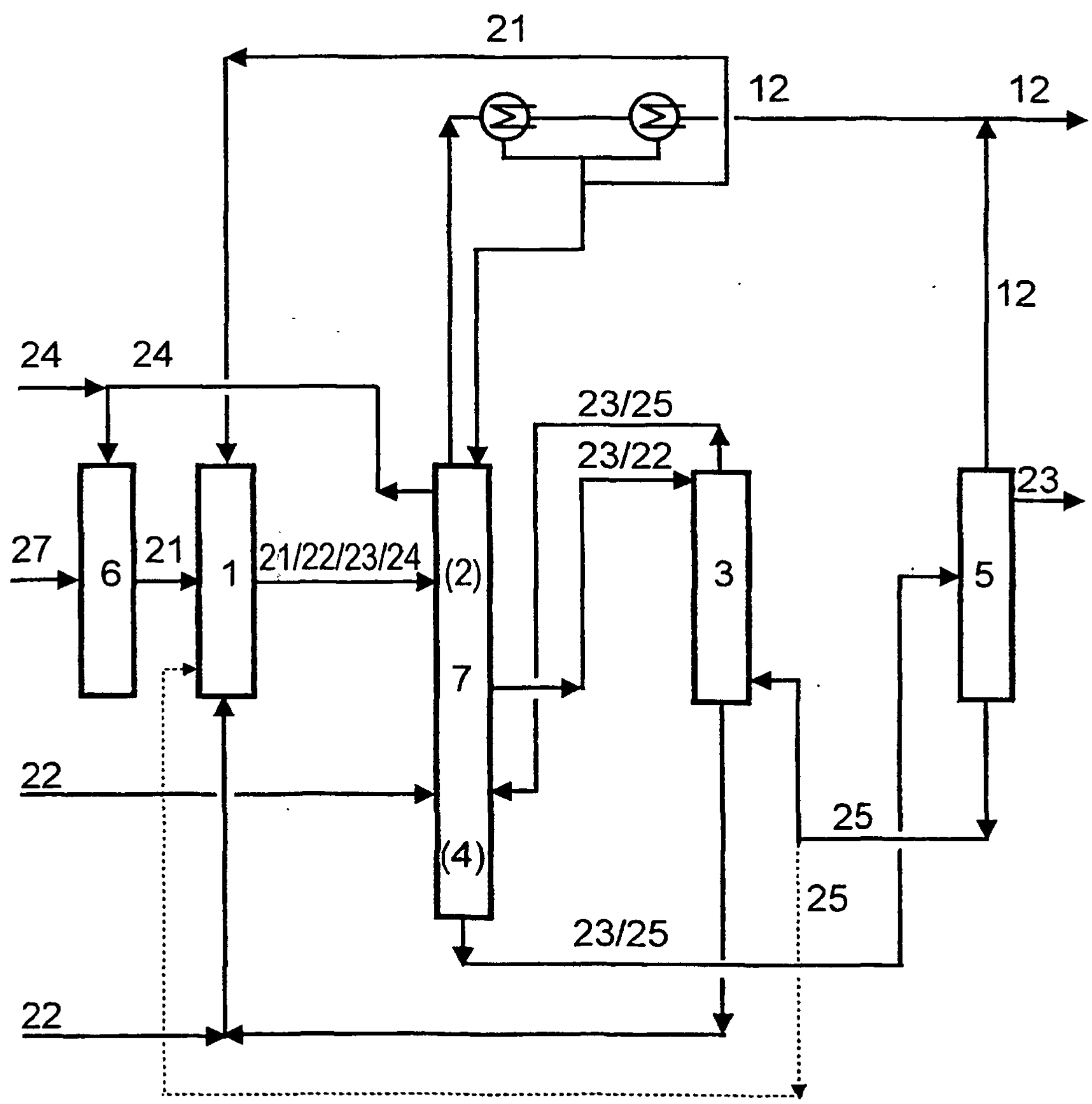
21
12
12
12
24
24
23/25
23/22
23
27
6
21
1
21/22/23/24
(2)
3
5
7
22
25
(4)
25
23/25
22

FIG.3

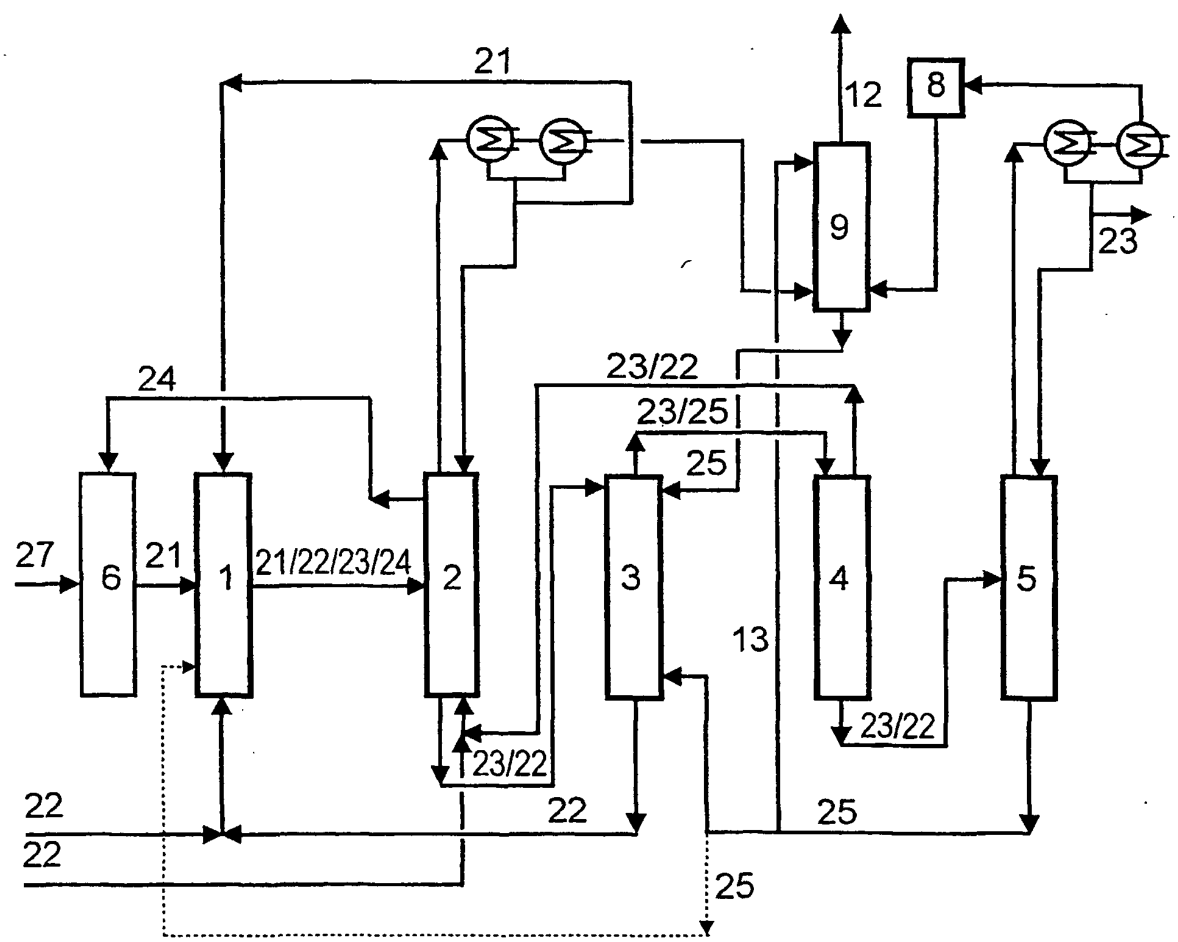
21
12
8
9
23
24
23/22
23/25
25
27
6
21
1
21/22/23/24
2
3
4
5
13
23/22
23/22
22
22
25
22
25

FIG.4

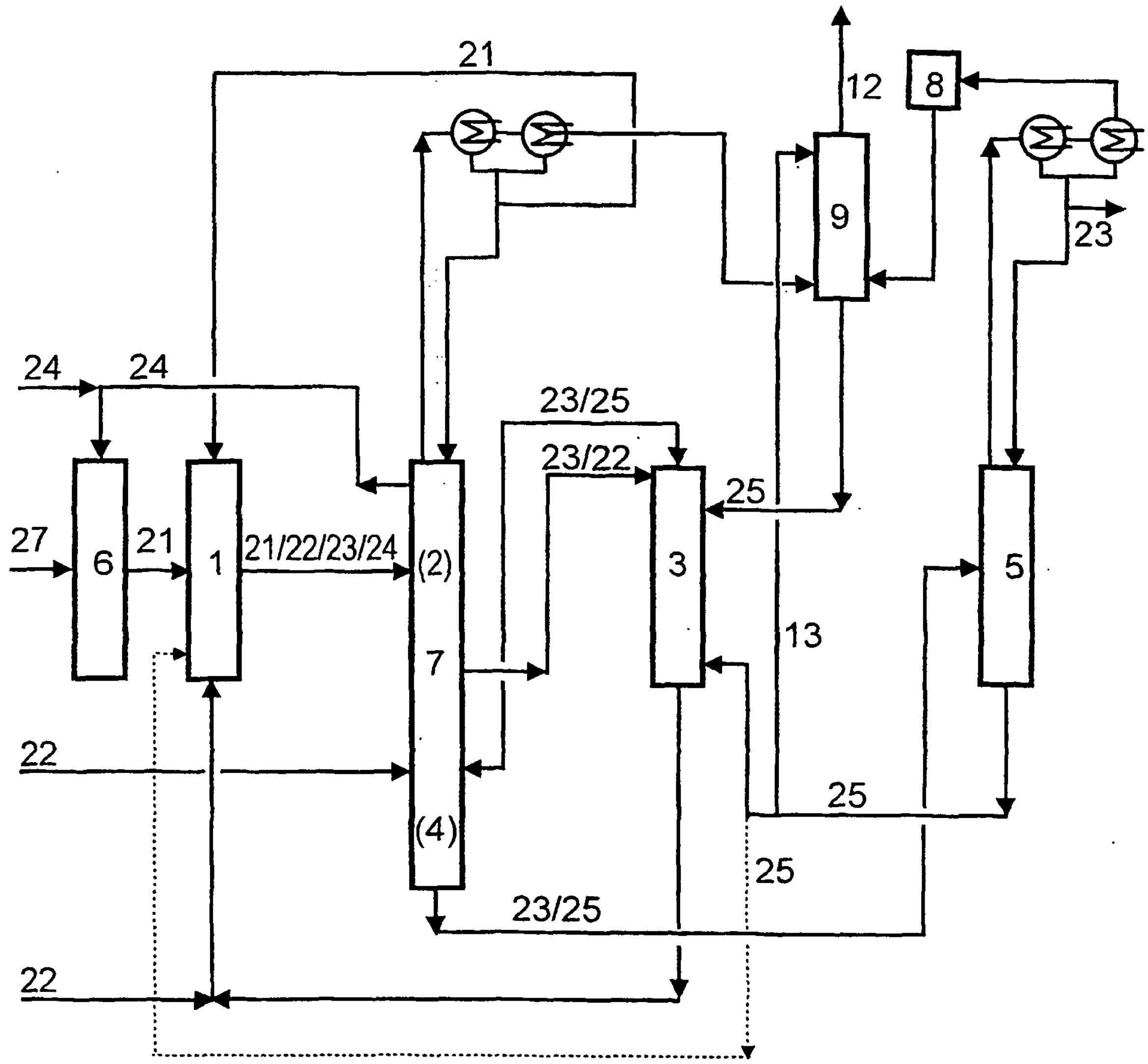
21
12
8
9
23
24
24
23/25
23/22
25
27
6
21
1
21/22/23/24
(2)
3
5
7
13
22
25
(4)
25
23/25
22